(19) Europäisches Patentamt
European Patent Office
Office européen des brevets

(11) Veröffentlichungsnummer: **0 052 853**
**A1**

# (12) EUROPÄISCHE PATENTANMELDUNG

(21) Anmeldenummer: **81109738.5**

(22) Anmeldetag: **17.11.81**

(51) Int. Cl.³: **C 07 C 97/24**
C 07 D 295/10, C 07 D 207/08
C 07 D 211/32, C 07 D 265/30
A 61 K 31/135, A 61 K 31/40
A 61 K 31/445, A 61 K 31/495
A 61 K 31/535, A 61 K 31/55

(30) Priorität: **24.11.80 DE 3044072**
**07.05.81 DE 3117984**

(43) Veröffentlichungstag der Anmeldung:
**02.06.82 Patentblatt 82/22**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt/Main 80(DE)**

(72) Erfinder: **Winkelmann, Erhardt, Dr.**
**Brunhildenweg 5**
**D-6233 Kelkheim (Taunus)(DE)**

(72) Erfinder: **Raether, Wolfgang, Dr.**
**Falkensteinstrasse 6**
**D-6072 Dreieich(DE)**

(72) Erfinder: **Rolly, Heinrich, Dr.**
**Am Tulpengarten 18**
**D-6233 Kelkheim (Taunus)(DE)**

## (54) Bis-Aminomethyl-anthrachinon-Derivate, Verfahren zu ihrer Herstellung, sie enthaltende Mittel und ihre Verwendung.

(57) Es werden neue Bis- (aminomethyl)-anthrachinon-Derivate der Formel

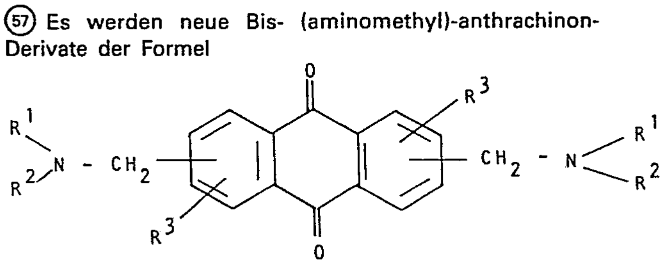

und ihre Salze mit physiologisch verträglichen Säuren beschrieben, sowie Verfahren zu ihrer Herstellung. Die neuen Verbindungen sind wirksam gegen Protozoen, Viren und Krebserkrankungen.

In der obigen Formel bedeuten $R^1$ und $R^2$, die gleich oder verschieden sind, Wasserstoff, geradkettiges oder verzweigtes Alkyl mit ein bis vier Kohlenstoffatomen, oder es bedeuten $R^1$ und $R^2$ zusammen eine Alkylenkette mit vier, fünf oder sechs Kohlenstoffatomen, die mit dem Stickstoffatom einen fünf-, sechs- oder siebengliedrigen heterocyclischen Ring bilden, der ein weiteres Heteroatom aus der Gruppe Stickstoff, Sauerstoff und Schwefel enthalten kann und seinerseits durch geradkettiges oder verzweigtes Alkyl mit ein bis vier Kohlenstoffatomen substituiert sein kann, und $R^3$ bedeutet Wasserstoff oder die Hydroxylgruppe.

EP 0 052 853 A1

Croydon Printing Company Ltd.

# Bis-Aminomethyl-anthrachinon-Derivate, Verfahren zu ihrer Herstellung, sie enthaltende Mittel und ihre Verwendung

2,6-Diamino-anthrachinone sind nach DE-OS 25 38 878 und Anthrachinon-bis-amidine sind nach DE-OS 25 21 357 zur Bekämpfung von Protozoenerkrankungen wie Amöbiasis bekannt. 1-Hydroxy-2-aminomethyl-anthrachinone sind nach Liebigs Ann. Chem. 1975, Seite 972, bekannt; sie haben jedoch keine Wirkung gegenüber Protozoen.

Gegenstand der Erfindung sind Bis-Aminomethyl-anthrachinon-Derivate der Formel I

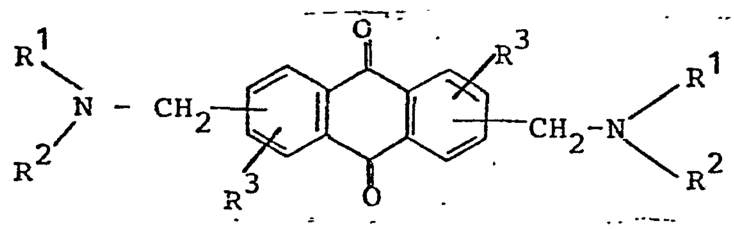

(I)

und ihre Salze mit physiologisch verträglichen Säuren, worin $R^1$ und $R^2$ gleich oder verschieden sind und Wasserstoff, geradkettiges oder verzweigtes Alkyl mit ein bis vier Kohlenstoffatomen, wie Methyl, Äthyl, Propyl, Isopropyl, Butyl, Isobutyl, oder $R^1$ und $R^2$ zusammen eine Alkylenkette mit vier, fünf oder sechs Kohlenstoffatomen die mit dem Stickstoffatom einen fünf-, sechs- oder siebengliedrigen heterocyclischen Ring bilden, der ein weiteres Heteroatom aus der Gruppe Stickstoff, Sauerstoff und Schwefel enthalten kann und seinerseits durch geradkettiges oder verzweigtes Alkyl mit ein bis vier Kohlenstoffatomen substituiert sein kann, wie Pyrrolidin, 2-Methyl-pyrrolidin, 2,5-Dimethyl-pyrrolidin, Piperidin, 2-Methyl-piperidin, 2,6-Dimethyl-piperidin, 3-Methyl-piperidin, 4-Methyl-piperidin, 4-Dimethylamino-piperidin, 4-Piperidino-piperidin Morpholin, 2-Methyl-morpholin, 2,6-Dimethyl-morpholin, Piperazin, N-Methyl-piperazin, N-Äthyl-piperazin, N-Propyl-piperazin, N-Butyl-piperazin, N-2-Hydroxyäthyl-piperazin, N-3-Hydroxypropyl-piperazin, N-Methyl-hexahydropyrimidin, N-Methyl-homopiperazin, Hexamethylenimin oder Thiomorpholin, bedeuten, und worin $R^3$ für Wasserstoff oder die Hydroxylgruppe steht.

0052853

Bevorzugt sind Verbindungen der Formel I, in welcher sich die beiden Aminomethyl-Reste in 2,6- oder 2,7-Position und die beiden Hydroxylgruppen $R^3$ sich in 1,5- oder 1,8-position am Anthrachinongerüst befinden.

Bevorzugt sind weiter Verbindungen der Formel I, in welcher beide substituierten Amino-Gruppen eine Dimethylamino-, Diäthylamino-, Pyrrolidino-, Morpholino- oder N-Alkylpiperazino-gruppe darstellen.

Gegenstand der Erfindung ist auch ein Verfahren zur Herstellung von Bis-Aminomethyl-anthrachinon-Derivaten der Formel I sowie von deren Salzen, das dadurch gekennzeichnet ist, daß man

a) ein substituiertes Dimethyl-anthrachinon der Formel II

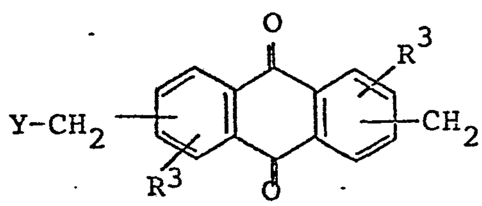

(II)

worin $R^3$ für Wasserstoff oder die Hydroxylgruppe steht und Y ein Halogenatom wie Chlor, Brom, Jod, eine Acyloxygruppe oder eine Arylsulfonyloxygruppe, insbesondere eine Phenylsulfonyloxygruppe, bedeutet, mit einem Amin der Formel III

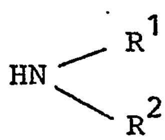

(III)

worin $R^1$ und $R^2$ die zu Formel I erwähnten Bedeutungen haben, umsetzt und gegebenenfalls durch Zugabe einer physiologisch verträglichen Säure in ein Salz überführt, oder

b) ein Anthrachinon-dicarbonsäureamid der Formel IV

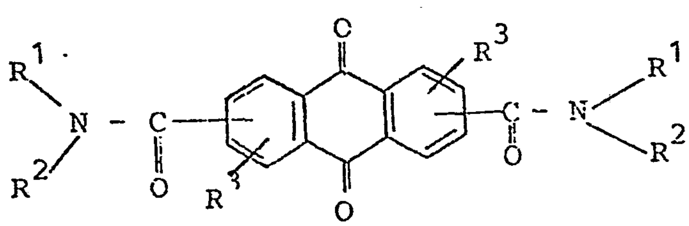

(IV)

worin $R^1$ und $R^2$ die zu Formel I erwähnten Bedeutungen haben und $R^3$ für Wasserstoff steht, reduziert und die als Zwischenprodukt erhaltenen Anthrahydrochinon-Derivate zu Anthrachinon-Derivaten der Formel I oxydiert und gegebenenfalls durch Zugabe einer physiologisch verträglichen Säure in ein Salz überführt, oder

c) ein Anthrachinon der Formel V

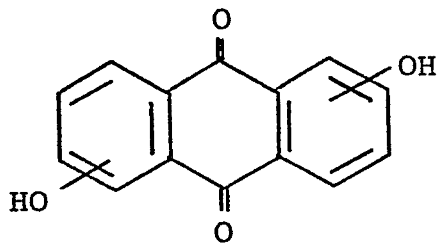

(V)

zu einem Anthrahydrochinon reduziert, danach - vorteilhaft ohne Isolierung - mit Formaldehyd und einem Amin der Formel III umsetzt und das als Zwischenprodukt erhaltene Bis-aminomethyl-anthrahydrochinon-Derivat zu einem Anthrachinon-Derivat der Formel I oxydiert und diese gegebenenfalls durch Zugabe einer physiologisch verträglichen Säure in ein Salz überführt.

Die Herstellung der Verbindungen der Formel I nach Verfahren a) wird zweckmäßig mit äquivalenten Mengen der Ausgangsstoffe der Formel II (1 Mol) und der Formel III (2 Mol) in Gegenwart eines säurebindenden Mittels (2 Mol) oder auch durch Anwendung eines Überschusses des Amins der Formel III (4 Mol) durchgeführt.

Als Ausgangsstoffe der Formel II kommen beispielsweise in Frage 2,6- bzw. 2,7-Bis-Chlormethyl-,-Brommethyl-, -Jodmethyl-, -Acetoxymethyl-, -Benzoyloxymethyl-, -Methansulfonyloxymethyl-, -Benzolsulfonyloxymethyl- und -4-Toluolsulfonyloxymethyl-anthrachinon, sowie 1,5-Dihydroxy-2,6-bis-chlormethyl-, -brommethyl-, -jodmethyl-, -acetoxymethyl-, -benzoyloxymethyl-, -methansulfonyloxymethyl-, -benzolsulfonyloxymethyl- und -4-toluolsulfonyloxymethyl-anthrachinon, sowie 1,8-Dihydroxy-2,7-bis-chlormethyl-, -brommethyl-, -jodmethyl-, -acetoxymethyl-, benzoyloxymethyl-, -methansulfonyloxymethyl-, -benzolsulfonyloxymethyl- und -4-toluolsulfonyl-oxymethyl-anthrachinon.

Bevorzugt sind einerseits 2,6- bzw. 2,7-Bis-Brommethyl-anthrachinone ($R^3$ = H). Diese Verbindungen können erhalten werden durch Bromierung von 2,6- bzw. 2,7-Dimethylanthrachinon z.B. mittels N-Bromsuccinimid. 2,6- bzw. 2,7-Dimethylanthrachinon können nach der deutschen Patentschrift 494 433 durch Diensynthese aus Benzochinon und Isopren dargestellt werden.

Bevorzugt sind andererseits 1,5-Dihydroxy-2,6-bis-(chlormethyl)-anthrachinon und 1,8-Dihydroxy-2,7-bis-(chlormethyl)-anthrachinon ($R^3$ = OH). Diese Verbindungen können erhalten werden durch Chlorierung von 1,5-Dihydroxy-2,6-bis-(hydroxymethyl)-anthrachinon bzw. 1,8-Dihydroxy-2,7-bis-(hydroxymethyl)-anthrachinon mittels Thionylchlorid. Die Darstellung von 1,5-Dihydroxy-2,6-bis-(hydroxymethyl)-anthrachinon und 1,8-Dihydroxy-2,7-bis-(hydroxymethyl)-anthrachinon ist in Liebigs Ann. Chem. 1975, Seite 972 beschrieben und erfolgt durch Hydroxymethylierung von 1,5-Dihydroxy- bzw. 1,8-Dihydroxy-anthrahydrochinon mit Formaldehyd in alkalisch-wäßriger Lösung und anschließender Oxydation mit Luftsauerstoff zum entsprechenden Anthrachinon.

Als Ausgangsstoffe der Formel III kommen beispielsweise in Frage Ammoniak oder Ammoniakbildner, wie Ammoniumcarbonat, Harnstoff, Urotropin, ferner Dimethylamin, Diäthylamin, Dipropylamin, Diisopropylamin, Dibutylamin, Diisobutylamin, Methyläthylamin, Methylpropylamin, Methylbutylamin, Pyrrolidin, 2-Methyl-pyrrolidin, 2,5-Dimethyl-pyrrolidin, Piperidin, 2-Methyl-piperidin, 2,6-Dimethyl-piperidin, 3-Methyl-piperidin, 4-Methyl-piperidin, 4-Dimethylamino-piperidin, 4-Piperidino-piperidin, Morpholin, 2-Methyl-morpholin, 2,6-Dimethyl-morpholin, Piperazin, N-Methyl-piperazin, N-Äthyl-piperazin, N-Propyl-piperazin, N-Butyl-piperazin, N-2-Hydroxyäthyl-piperazin, N-3-Hydroxypropyl-

piperazin, N-Methyl-hexahydropyrimidin, N-Methyl-homopiperazin, Hexamethylenimin und Thiomorpholin.

Die Ausgangsstoffe der Formel III sind bekannt.

Als säurebindende Mittel kommen beispielsweise in Frage Basen wie Triäthylamin, N-Äthylmorpholin, Pyridin, Chinolin, Alkali-, Erdalkali-carbonate, -bicarbonate.

Die Herstellung wird vorteilhaft in einem Lösungs- oder Verteilungsmittel durchgeführt. Als Lösungs- oder Verteilungsmittel kommen beispielsweise in Frage Alkohole, wie Methanol, Äthanol, Propanol, Butanol, Methoxyäthanol, Äthoxyäthanol, Ketone wie Aceton, Methyläthylketon, Diäthylketon, Methylisobutylketon, Amide wie Dimethylformamid, Dimethylacetamid, N-Methylpyrrolidon, Tetramethylharnstoff, Hexamethylphosphorsäuretriamid, Äther wie Dipropyläther, Dibutyläther, Äthylenglycoldimethyläther, -diäthyläther, Diäthylenglycoldimethyläther, Tetrahydrofuran, Dioxan, gegebenenfalls chlorierte, aliphatische und aromatische Kohlenwasserstoffe, wie Methylenchlorid, Chloroform, Dichloräthan, Tetrachloräthan, Benzol, Toluol, Xylol, Chlorbenzol, Dichlorbenzol, ferner Dimethylsulfoxid, Tetramethylensulfon, Acetonitril.

Die Reaktionszeiten betragen wenige Minuten bis einige Stunden. Die Reaktionstemperaturen liegen zwischen 0 und 100°C, vorteilhaft falls $R^3$ Wasserstoff ist zwischen 60 und 80°C, falls $R^3$ die Hydroxylgruppe ist, zwischen 10° und 30°C.

Die Herstellung der Verbindungen der Formel I nach Verfahren b) wird durch Umsetzung eines Reduktionsmittels mit einem Anthrachinon-dicarbonsäureamid der Formel IV durchgeführt.

Als Ausgangsstoffe der Formel IV kommen beispielsweise in Frage Anthrachinon-2,6-, bzw. -2,7-dicarbonsäureamide des Ammoniak, Dimethylamin, Diäthylamin, Dipropylamin, Diisopropylamin, Dibutylamin, Diisobutylamin, Methyläthylamin, Methylpropylamin, Methylbutylamin, Pyrrolidin, 2-Methyl-pyrrolidin, 2,5-Dimethyl-pyrrolidin, Piperidin, 2-Methyl-piperidin, 2,6-Dimethyl-piperidin, 3-Methyl-piperidin, 4-Methyl-piperidin, 4-Dimethylamino-piperidin, 4-Piperidino-piperidin, Morpholin, 2-Methyl-morpholin, 2,6-Dimethyl-morpholin, Piperazin, N-Methyl-piperazin, N-Äthyl-piperazin, N-Propyl-piperazin, N-Butyl-piperazin, N-2-Hydroxyäthyl-piperazin, N-3-Hydroxypropyl-piperazin, N-Methyl-hexahydropyrimidin, N-Methyl-homopiperazin, Hexamethylenimin und Thiomorpholin.

Die Ausgangsstoffe der Formel IV können erhalten werden durch Umsetzung von Anthrachinon-2,6- bzw. -2,7-dicarbonsäureester oder Anthrachinon-2,6-, bzw -2,7-dicarbonsäurechloride mit entsprechenden Aminen.

Die den Anthrachinon-2,6- bzw. -2,7-carbonsäurederivaten zugrunde liegenden Carbonsäuren können nach US-Patent 3 939 276 durch Oxydation von 2,6- bzw. 2,7-Dimethyl-anthrachinon mittels Chromsäure erhalten werden.

Als Reduktionsmittel für Verfahren b) kommen vorzugsweise in Frage Metallhydride, wie Lithiumaluminiumhydrid oder Natriumaluminiumbisalkoxydihydrid. Die Metallhydride können in äquivalenten Mengen angewendet werden, z.B. auf 1 Mol Anthrachinon-dicarbonsäureamid der Formel IV 1,5 Mol Lithiumalanat. Vorteilhafterweise empfiehlt sich jedoch die Anwendung eines Überschusses an Reduktionsmittel bis 100 %.

Die Reduktionen werden vorteilhaft in einem Lösungs- oder Verteilungsmittel durchgeführt. Als Lösungs- oder Verteilungsmittel kommen beispielsweise in Frage Äther wie Diäthyläther, Dipropyläther, Dibutyläther, Äthylen-glycoldimethyläther, -diäthyläther, Diäthylenglycoldimethyl-äther, Tetrahydrofuran, Dioxan. Als Lösungsvermittler können manchmal mit Vorteil Pyridin oder N-Äthylmorpholin zugesetzt werden.

Die Reduktionszeiten betragen wenige Minuten bis einige Stunden. Die Reduktionstemperaturen liegen zwischen 0 und 100°C, vorteilhaft zwischen 25 und 60°C.

Die nach der Reduktion der Anthrachinon-dicarbonsäureamide der Formel IV als Zwischenprodukte anfallenden Anthrahydrochinone werden durch Behandeln mit einem Oxydationsmittel in Anthrachinon-Derivate der Formel I umgewandelt. Als Oxydationsmittel wird vorteilhafterweise Luftsauerstoff verwendet. Zur Oxydation wird zweckmäßig durch eine wässrige oder alkoholisch-wässrige Suspension des Anthrahydrochinons Luft geleitet.

Die Herstellung der Verbindungen der Formel I nach Verfahren c) wird zweckmäßig mit äquivalenten Mengen der Ausgangsstoffe der Formel V (1 Mol) und der Formel III (2 Mol) und Formaldehyd (2 Mol) oder auch durch Anwendung eines Überschusses des Amins der Formel III und des Formaldehyds durchgeführt.

Die Reduktion der Dihydroxy-anthrachinone der Formel V zu Dihydroxy-anthrahydrochinonen erfolgt vorteilhaft in alkalisch-wäßriger Lösung bei Raumtemperatur unter Stickstoff als Schutzgas. Als Reduktionsmittel verwendet man vorteilhaft Natriumdithionit ($Na_2S_2O_4$) in äquimolarer Menge (1:1) zum Dihydroxyanthrachinon. Weiterhin können verwendet werden Metallhydride wie Lithiumaluminiumhydrid, katalytisch durch Raney-Nickel, Palladium oder Platin erregter Wasserstoff oder nascierender Wasserstoff, z.B. als Zinn oder Zink mit Mineralsäure, insbesondere Salzsäure. Die anschließende Mannich-Reaktion (Amidomethylierung) wird zweckmäßig durch gleichzeitige Zugabe von Formaldehyd und Amin der Formel III zur alkalisch-wäßrigen Reduktionslösung, die das Dihydroxy-anthrahydrochinon enthält, durchgeführt. Formaldehyd und Amin werden dabei vorteilhaft im Überschuß (bis 100 %) angewendet. Formaldehyd wird zweckmäßig in wäßriger Lösung angewendet. Die Mannich-Reaktion erfolgt durch Rühren der Reaktionsmischung bei

Raumtemperatur, gegebenenfalls unter Kühlung. Die Reaktionszeiten betragen wenige Minuten bis zu 1 Stunde. Die anschließende Oxydation der so erhaltenen Anthrahydrochinone erfolgt vorteilhaft durch Durchleiten von Luftsauerstoff durch die Reaktionslösung bei Raumtemperatur. Die Oxydation kann auch durch Sauerstoff, Wasserstoffperoxid oder Peroxysäuren wie Peroxybenzoesäure erfolgen. Nach dem Neutralisieren der Reaktionslösung, z.B. mit einer Mineralsäure wie Salzsäure, oder durch verdünnte Alkalien wie Natronlauge oder Natriumcarbonatlösung fallen die Endprodukte der Formel I in kristalliner Form an.

Die Isolierung der Verbindungen der Formel I, wie sie nach Verfahren a), b) oder c) erhalten werden, erfolgt nach üblichen Methoden durch Abdestillieren der verwendeten Lösungsmittel oder durch Verdünnen der Reaktionslösung mit Wasser und Absaugen des Endproduktes.

Gegebenenfalls kann eine Reinigung der Endprodukte durch Umkristallisieren aus einem geeigneten Lösungsmittel wie Äthanol, Isopropanol oder Glykolmonomethyläther oder aus einem Lösungsmittelgemisch wie Dimethylformamid mit einem der vorgenannten erfolgen.

Die neuen Verbindungen der Formel I können, wenn gewünscht, durch Zusetzen einer physiologisch verträglichen Säure in das entsprechende Salz übergeführt werden. Als physiologisch verträgliche Säuren kommen beispielsweise in Frage Halogenwasserstoffsäuren, insbesondere Salzsäure, ferner Schwefelsäure, Phosphorsäure, oder organische Säuren wie Essigsäure, Milchsäure, Weinsäure, Zitronensäure, Bernsteinsäure oder Glutarsäure.

Die Bis-Aminomethylanthrachinon-Derivate der Formel I sind gut verträglich und eignen sich zur Bekämpfung von Protozoenerkrankungen bei Mensch und Tier. Insbesondere wirken diejenigen Verbindungen der Formel I, in denen $R^3$ Wasser-

stoff ist, bevorzugt gegen Amöbiasis, die durch Infektionen mit Entamoeba histolytica hervorgerufen wird. Außerdem entfalten diese Verbindungen eine direkte oder durch ihre interferoninduzierende Eigenschaft indirekte Wirkung gegen Influenza- und Encephalomyocarditis-Viren. Durch ihre interferon-induzierende Wirkung sind sie geeignet zur Bekämpfung von Virus- und Krebserkrankungen.

Weiterhin wirken insbesondere diejenigen Verbindungen der Formel I, in denen $R^3$ die Hydroxylgruppe ist, bevorzugt gegen Trichomoniasis, wie sie durch Infektionen mit Trichomonas fetus oder Trichomonas vaginalis hervorgerufen wird.

Wirkungen sowohl gegen Amöben als auch gegen Trichomonaden sind besonders ausgeprägt z. B. bei 1,8-Dihydroxy-2,7-bis-(dimethylaminomethyl)-anthrachinon-9,10.

Die Anwendung der neuen Verbindungen kann oral, parenteral oder lokal erfolgen, z.B. nasal bei der Behandlung der Influenza oder vaginal bei der Behandlung der Trichomoniasis.

Die orale Anwendung erfolgt in pharmazeutisch üblichen Zubereitungen, z.B. in Form von Tabletten oder Kapseln. Pro Tagesdosis werden etwa 10 bis 1000 mg des Wirkstoffes in Mischung mit einem gebräuchlichen Trägerstoff und/oder Konstituens appliziert. Die parenterale Anwendung erfolgt in Form von Lösungen, insbesondere in Wasser oder in physiologischer Kochsalzlösung. Die nasale Anwendung erfolgt in Form von Sprays, bei welchen eine geeignete Lösung des Verfahrenserzeugnisses, z.B. in wäßrige Lösung eines Hydrochlorids, durch ein Treibmittel fein zerstäubt wird. Die vaginale Applikation erfolgt in Tabletten oder in Form von Pasten, Gelees oder Globuli.

Herstellungsbeispiele:

Beispiel 1 (Verfahren a))

Beispiel 1.1

2,6-Bis-(N-Methylpiperazinomethyl)-anthrachinon-9,10

39,4 g (0,1 Mol) 2,6-Bis-Brommethyl-anthrachinon-9,10 (Fp. 316°C) werden in 500 ml Äthanol suspendiert, 44 g (0,44 Mol) N-Methylpiperazin werden zugegeben und die Reaktionsmischung 1,5 Stunden unter Rückfluß erhitzt. Dabei geht 2,6-Bis-Brommethyl-anthrachinon-9,10 in Lösung. Danach wird die Lösung unter vermindertem Druck zur Trockene eingedampft, der feste Rückstand mit Wasser angerührt, abfiltriert, mit Wasser gewaschen und aus Äthanol unter Zusatz von Aktivkohle umkristallisiert. Man erhält so 33 g (76 % der Theorie) 2,6-Bis-(N-piperazinomethyl)-anthrachinon-9,10 in Form von gelblichen Kristallen vom Fp. 202°C.

Das als Ausgangsstoff verwendete 2,6-Bis-Brommethyl-anthrachinon-9,10 erhält man durch Umsetzung von 2,6-Dimethyl-anthrachinon-9,10 mit N-Bromsuccinimid in 80%iger Ausbeute in Form von gelben Kristallen mit einem Fp. 316°C (Zersetzung).

Analog erhält man aus 2,7-Dimethyl-anthrachinon-9,10 mit N-Bromsuccinimid, 2,7-Bis-Brommethyl-anthrachinon-9,10 vom Fp. 265°C.

Nach dem in Beispiel 1 beschriebenen Verfahren können durch Umsetzung von 2,6-Bis-Brommethyl-anthrachinon-9,10 und entsprechenden Aminen der Formel III erhalten werden:

Beispiel 1.2 bis 1.33

2,6-Bis-(Aminomethyl)-anthrachinon-9,10x2 HCl, Fp. 300°C

2,6-Bis-(Dimethylaminomethyl)-anthrachinon-9,10x2 HCl, Fp. 300°C, Base 155°C

2,6-Bis-(Diäthylaminomethyl)-anthrachinon-9,10 Fp.94°C
2,6-Bis-(Dipropylaminomethyl)-anthrachinon-9,10 Fp.40°C
2,6-Bis-(Dibutylaminomethyl)-anthrachinon-9,10 Oel
2,6-Bis-(Methyläthylaminomethyl)-anthrachinon-9,10
2,6-Bis-(Pyrrolidinomethyl)-anthrachinon-9,10 Fp.156°C
2,6-Bis-(2,5-Dimethylpyrrolidinomethyl)-anthrachinon-9,10
2,6-Bis-(Piperidinomethyl)-anthrachinon-9,10 Fp.184°C
2,6-Bis-(2-Methylpiperidinomethyl)-anthrachinon-9,10 Fp.193°C
2,6-Bis-(2,6-Dimethylpiperidinomethyl)-anthrachinon-9,10 Fp.160°C
2,6-Bis-(Morpholinomethyl)-anthrachinon-9,10 Fp.224°C
2,6-Bis-(2,6-Dimethylmorpholinomethyl)-anthrachinon-9,10
2,6-Bis-(4-Piperidinopiperidinomethyl)-anthrachinon-9,10 Fp.181°C
2,6-Bis-(N-Äthylpiperazinomethyl)-anthrachinon-9,10 Fp. 165°C
2,6-Bis-(N-2-Hydroxyäthylpiperazinomethyl)-anthrachinon-9,10 Fp.168°C
2,6-Bis-(N-Methylhomopiperazinomethyl)-anthrachinon-9,10 Fp. 200°C
2,6-Bis-(Hexamethyleniminomethyl)-anthrachinon-9,10 Fp. 186°C

Nach dem in Beispiel 1.1 beschriebenen Verfahren können durch Umsetzung von 2,7-Bis-Brommethyl-anthrachinon-9,10 und entsprechenden Aminen der Formel III erhalten werden:

2,7-Bis-(Aminomethyl)-anthrachinon-9,10
2,7-Bis-(Dimethylaminomethyl)-anthrachinon-9,10 Fp.96°C
2,7-Bis-(Diäthylaminomethyl)-anthrachinon-9,10 Fp. 68°C
2,7-Bis-(Dipropylaminomethyl)-anthrachinon-9,10 Oel
2,7-Bis-(Dibutylaminomethyl)-anthrachinon-9,10
2,7-Bis-(Methyläthylaminomethyl)-anthrachinon-9,10
2,7-Bis-(Pyrrolidinomethyl)-anthrachinon-9,10 Fp. 120°C
2,7-Bis-(2,5-Dimethylpyrrolidinomethyl)-anthrachinon-9,10
2,7-Bis-(Piperidinomethyl)-anthrachinon-9,10 Fp. 166°C
2,7-Bis-(2-Methylpiperidinomethyl)-anthrachinon-9,10
2,7-Bis-(2,6-Dimethylpiperidinomethyl)-anthrachinon-9,10
2,7-Bis-(4-piperidinopiperidinomethyl)-anthrachinon-9,10 Fp.167°C
2,7-Bis-(Morpholinomethyl)-anthrachinon-9,10 Fp.190°C
2,7-Bis-(2,6-Dimethylmorpholinomethyl)-anthrachinon-9,10

| | |
|---|---|
| 2,7-Bis-(N-Methylpiperazinomethyl)-anthrachinon-9,10 | Fp. 138°C |
| 2,7-Bis-(N-Äthylpiperazinomethyl)-anthrachinon-9,10 | Fp. 120°C |
| 2,7-Bis-(N-2-Hydroxyäthylpiperazinomethyl)-anthrachinon-9,10 | Fp. 109°C |
| 2,7-Bis-(N-Methylhomopiperazinomethyl)-anthrachinon-9,10 | |
| 2,7-Bis-(Hexamethyleniminomethyl)-anthrachinon-9,10 | |

Beispiel 2 (Verfahren a))

2.1.) 1,5-Dihydroxy-2,6-bis-(dimethylaminomethyl)-anthrachinon-9,10

33,7 g (0,1 Mol) 1,5-Dihydroxy-2,6-bis-(chlormethyl)-anthrachinon-9,10 werden in 1 Liter Äthylenglycoldimethyläther suspendiert und gasförmiges Dimethylamin eingeleitet. In leicht exothermer Reaktion steigt die Innentemperatur von 25 auf 50°C, und das Anthrachinon geht in Lösung. Nach 30 Minuten wird das Einleiten beendet, die Reaktionslösung im Vakuum eingedampft, der Rückstand mit Wasser angerührt, mit verd. Natronlauge neutralisiert, abgesaugt, mit Wasser gewaschen und aus Alkohol umkristallisiert.

Man erhält so 30,0 g (85 % der Theorie) 1,5-Dihydroxy-2,6-bis-(dimethylaminomethyl)-anthrachinon-9,10 in Form von gelben Kristallen mit dem Fp. 154°C.

Durch Zusatz einer äquimolaren Menge alkoholischer Salzsäure kann die Verbindung in das Di-Hydrochlorid überführt werden: Gelbliche Kristalle vom Fp. >300°C.

Das als Ausgangsstoff verwendete 1,5-Dihydroxy-2,6-bis-(chlor-methyl)-anthrachinon-9,10 kann durch Umsetzung von 1,5-Dihydroxy-2,6-bis-(hydroxymethyl)-anthrachinon-9,10

mittels Thionylchlorid (10fache Menge $SOCl_2$, ohne Lösungsmittel, 4 Stunden bei 80°C) dargestellt werden: Goldgelbe Kristalle (aus Toluol umkristallisiert) vom Fp. 236°C.

Das bei dieser Umsetzung verwendete 1,5-Dihydroxy-2,6-bis-(hydroxy-methyl)-anthrachinon-9,10 wird nach Liebigs Ann. Chem. 1975, Seite 983, aus 1,5-Dihydroxy-anthrachinon-9,10 und Formaldehyd dargestellt: Gelbe Kristalle (aus Toluol) vom Fp. 245°C.

2.2.) In gleicher Verfahrensweise erhält man aus 1,8-Dihydroxy-2,7-bis-(chlormethyl)-anthrachinon-9,10 und Dimethylamin das entsprechende 1,8-Dihydroxy-2,7-bis-(dimethylaminomethyl)-anthrachinon-9,10 vom Fp. 145°C in guten Ausbeuten. (2 HCl-Salze Fp. >300°C). Der Ausgangsstoff 1,8-Dihydroxy-2,7-bis-(chlormethyl)-anthrachinon-9,10 (Fp. 256°C) wird durch Umsetzung von 1,8-Dihydroxy-2,7-bis-(hydroxymethyl)-anthrachinon-9,10 mit Thionylchlorid dargestellt. 1,8-Dihydroxy-2,7-bis-(hydroxymethyl)-anthrachinon-9,10 (Fp. 268°C) ist nach Liebigs Ann.Chem. 1975, Seite 983 bekannt.

In Analogie zu dem in Beispiel 2.1 beschriebenen Verfahren a) werden durch Umsetzung von 1,5-Dihydroxy-2,6-bis-(chlormethyl)-anthrachinon-9,10 bzw. 1,8-Dihydroxy-2,7-bis-(chlormethyl)-anthrachinon-9,10 mit entsprechenden Aminen der Formel III erhalten:

Beispiele 2.3 bis 2.18

1,5-Dihydroxy-2,6-bis-(aminomethyl)-anthrachinon-9,10 (2 HCl $>300^{\circ}C$)

1,5-Dihydroxy-2,6-bis-(diäthylaminomethyl)-anthrachinon-9,10 Fp. $96^{\circ}C$

1,5-Dihydroxy-2,6-bis-(methyläthylaminomethyl)-anthrachinon-9,10

1,5-Dihydroxy-2,6-bis-(pyrrodidinomethyl)-anthrachinon-9,10 Fp. $155^{\circ}C$

1,5-Dihydroxy-2,6-bis-(piperidinomethyl)-anthrachinon-9,10 Fp. $186^{\circ}C$

1,5-Dihydroxy-2,6-bis-(morpholinomethyl)-anthrachinon-9,10 Fp. $228^{\circ}C$ (2 HCl $>300^{\circ}C$)

1,5-Dihydroxy-2,6-bis-(N-methylpiperazinomethyl)-anthrachinon-9,10 Fp. $195^{\circ}C$

1,5-Dihydroxy-2,6-bis-(N-2-hydroxyäthylpiperazinomethyl)-anthrachinon-9,10

1,8-Dihydroxy-2,7-bis-(aminomethyl)-anthrachinon-9,10

1,8-Dihydroxy-2,7-bis-(diäthylaminomethyl)-anthrachinon-9,10

1,8-Dihydroxy-2,7-bis-(methyläthylaminomethyl)-anthrachinon-9,10

1,8-Dihydroxy-2,7-bis-(pyrrolidinomethyl)-anthrachinon-9,10

1,8-Dihydroxy-2,7-bis-(piperidinomethyl)-anthrachinon-9,10

1,8-Dihydroxy-2,7-bis-(morpholinomethyl)-anthrachinon-9,10 Fp. $247^{\circ}C$ (2 HCl $>300^{\circ}C$)

1,8-Dihydroxy-2,7-bis-(N-methylpiperazinomethyl)-anthrachinon-9,10 Fp. $216^{\circ}C$

1,8-Dihydroxy-2,7-bis-(N-2-hydroxyäthylpiperazinomethyl)-anthrachinon-9,10.

Beispiel 3 (Verfahren b))

Beispiel 3.1

2,6-Bis-(N-Methylpiperazinomethyl)-anthrachinon-9,10

4,6 g (0,01 Mol) Anthrachinon-9,10-dicarbonsäure-2,6-bis-(N-methylpiperazid) werden in 100 ml trockenem Tetrahydrofuran suspendiert. Unter Rühren und Temperaturen von 30-40°C (Kühlung) werden portionsweise 1,14 g (0,03 Mol = 100 % Überschuß) Lithiumaluminiumhydrid suspendiert in 50 ml Tetrahydrofuran eingetragen. Nach erfolgter Reaktion wird noch 3 Stunden unter Rühren auf 40-50°C erwärmt. Nach dem Abkühlen wird der Ansatz durch Zutropfen von Wasser unter Rühren und Kühlen hydrolysiert, die Lithiumaluminiumhydroxyde durch Absaugen abgetrennt, mit Tetrahydrofuran mehrfach gut gewaschen, die wässrige Tetrahydrofuranlösung unter kräftigem Durchleiten von Luft eingeengt. Dabei fällt das Endprodukt aus. Die Ausfällung wird durch Zugabe von Wasser vervollständigt, abgesaugt und aus Äthanol unter Kohlezusatz umkristallisiert. Man erhält so 2,6-Bis-(N-Methylpiperazinomethyl)-anthrachinon-9,10 in guter Ausbeute vom Fp. 202°C. Es ist identisch mit dem nach Verfahren a) Beispiel 1 erhaltenen Endprodukt.

Das als Ausgangsstoff verwendete Anthrachinon-9,10-dicarbonsäure-2,6-bis-(N-Methylpiperazid) vom Fp.248°C (Umwandlungspunkt Fp.226°C) erhält man durch Umsetzung von Anthrachinon-9,10-dicarbonsäurechlorid-2,6 vom Fp.210°C mit N-Methylpiperazin.

Beispiel 3.2

In analoger Weise wird durch Reduktion mit Lithiumaluminiumhydrid aus Anthrachinon-9,10-dicarbonsäure-2,7-bis-(N-Methylpiperazid) Fp. 145°C (aus Anthrachinon-9,10-dicarbonsäurechlorid-2,7 Fp.188°C und N-Methylpiperazin) 2,7-Bis-(N-Methylpiperazinomethyl)-anthrachinon-9,10 vom Fp.138°C in guter Ausbeute gewonnen.

Beispiel 4 (Verfahren c))

1,5-Dihydroxy-2,6-bis-(dimethylaminomethyl)-anthrachinon-9,10

24,0 g (0,1 Mol) 1,5-Dihydroxy-anthrachinon-9,10 werden in einer Lösung aus 16,0 g (0,4 Mol) Natriumhydroxid in 500 ml Wasser gelöst. Durch Einleiten von Stickstoff wird der Luftsauerstoff aus dem Reaktionsgefäß verdrängt und unter Rühren eine Lösung von 20,0 g (0,115 Mol) Natriumdithionit ($Na_2S_2O_4$) in 100 ml 2n-Natronlauge bei Raumtemperatur zugegeben. Nach wenigen Minuten ist die Reduktionsreaktion zum 1,5-Dihydroxy-anthrahydrochinon-9,10 beendet, was am Farbumschlag von weinrot nach orange erkennbar ist. Unter fortgesetztem Durchleiten von Stickstoff in mäßigem Gasstrom werden unter Rühren und gegebenenfalls geringer Kühlung mit Eiswasser bei 20 - 30°C 30,0 g (0.4 Mol = 100 % Überschuß) 40 %iger wäßriger Formaldehyd und danach 45,0 g (0,4 Mol = 100 % Überschuß) 40 %iges wäßriges Dimethylamin zugetropft. Danach wird noch 15 Minuten bei Raumtemperatur gerührt und sodann anstelle von Stickstoff für weitere 30 Minuten Luftsauerstoff bei Raumtemperatur durch die Lösung geleitet. Dabei erfolgt die Oxydation des gebildeten 1,5-Dihydroxy-2,6-bis-(dimethylaminomethyl)-anthrahydrochinon-9,10 zum 1,5-Dihydroxy-2,6-bis-(dimethyl-aminomethyl)-anthrachinon-9,10. Zur Ausfällung des Endproduktes wird unter Kühlung und Rühren durch Zutropfen von verdünnter, wäßriger Salzsäure die Reaktionslösung neutralisiert. Das kristallin anfallende Produkt wird abgesaugt, mit Wasser gewaschen und aus Alkohol umkristallisiert.

Man erhält so 21,0 g (59 % der Theorie) 1,5-Dihydroxy-2,6-bis-(dimethylaminomethyl)-anthrachinon-9,10 in Form von gelben Kristallen vom Fp. 154°C. Die Verbindung ist identisch mit der nach Verfahren a) in Beispiel 2.1 beschriebenen Verbindung.

Die bei Verfahren a) in den weiteren Beispielen 1.1 ff. und Beispielen 2.2 ff. genannten Verbindungen werden auch nach Verfahren c) erhalten.

Beispiel 5

1,8-Dihydroxy-2,7-bis-(dimethylaminomethyl)-anthrachinon-9.10

24,0 g (0,1 Mol) 1,8-Dihydroxy-anthrachinon-9,10 werden in einer Lösung aus 16,0 g (0,4 Mol) Natriumhydroxid in 500 ml Wasser gelöst. Durch Einleiten von Stickstoff wird der Luftsauerstoff aus dem Reaktionsgefäß verdrängt. Unter Rühren wird dann eine Lösung von 20,0 g (0,115 Mol) Natriumdithionit ($Na_2S_2O_4$) in 100 ml 2n-Natronlauge bei Raumtemperatur zugegeben. Nach wenigen Minuten ist die Reduktionsreaktion zum 1,8-Dihydroxy-anthrahydrochinon-9,10 beendet (Farbumschlag von weinrot nach orange). Unter weiterem Durchleiten von Stickstoff werden unter Rühren und gegebenenfalls Kühlung mit Eiswasser bei 20 - 30°C eine Mischung aus 30,0 g (0,4 Mol = 100 % Überschuß) 40 %igem wäßrigem Formaldehyd und 45,0 g (0,4 Mol = 100 % Überschuß) 40 %igem wäßrigem Dimethylamin zugetropft. Nach 15 Minuten Rühren bei Raumtemperatur wird anstelle von Stickstoff für weitere 30 Minuten Luftsauerstoff durch die Lösung geleitet. Dabei erfolgt die Oxydation des gebildeten 1,8-Dihydroxy-2,7-bis-(dimethyl-aminomethyl)-anthrachinon-9,10. Zur Ausfällung des Endproduktes wird unter Kühlung und Rühren durch Zutropfen von verdünnter, wäßriger Salzsäure die Reaktionslösung neutralisiert. Das kristallin anfallende Produkt wird abgesaugt, mit Wasser gewaschen und aus Alkohol umkristallisiert.

Man erhält so 22,2 g (63 % der Theorie) 1,8-Dihydroxy-2,7-bis-(dimethylaminomethyl)-anthrachinon-9,10 in Form von gelben Kristallen vom Fp. 145°C. Die Verbindung ist identisch mit der nach Verfahren a) in Beispiel 2.2 beschriebenen Verbindung.

0052853

PATENTANSPRÜCHE

HOE 80/F 267 K

1. Bis-Aminomethyl-anthrachinon-Derivate der Formel I

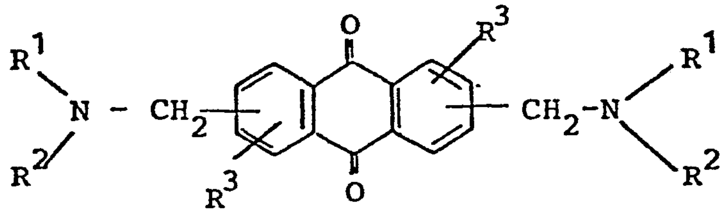

(I)

und ihre Salze mit physiologisch verträglichen Säuren, worin $R^1$ und $R^2$ gleich oder verschieden sind und Wasserstoff, geradkettiges oder verzweigtes Alkyl mit ein bis vier Kohlenstoffatomen, oder $R^1$ und $R^2$ zusammen eine Alkylenkette mit vier, fünf oder sechs Kohlenstoffatomen, die mit dem Stickstoffatom einen fünf-, sechs- oder siebengliedrigen heterocyclischen Ring bilden, der ein weiteres Heteroatom aus der Gruppe Stickstoff, Sauerstoff und Schwefel enthalten kann und seinerseits durch geradkettiges oder verzweigtes Alkyl mit ein bis vier Kohlenstoffatomen substituiert sein kann, bedeuten, und worin $R^3$ für Wasserstoff oder die Hydroxylgruppe steht.

2. Verfahren zur Herstellung von Bis-Aminomethyl-anthrachinon-Derivaten der Formel I in Anspruch 1 sowie von deren Salzen, dadurch gekennzeichnet, daß man

a) ein substituiertes Dimethyl-anthrachinon der Formel II

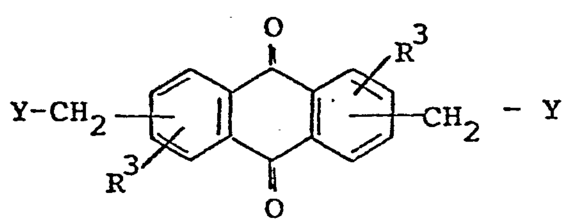

(II)

worin $R^3$ für Wasserstoff oder die Hydroxylgruppe steht und Y ein Halogenatom wie Chlor, Brom, Jod, eine Acyloxygruppe oder eine Arylsulfonyloxygruppe bedeutet, mit einem Amin der Formel III

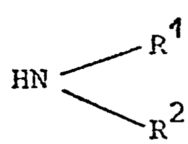

(III)

worin $R^1$ und $R^2$ die zu Formel I erwähnten Bedeutungen haben, umsetzt und gegebenenfalls durch Zugabe einer physiologisch verträglichen Säure in ein Salz überführt, oder

b) ein Anthrachinon-dicarbonsäureamid der Formel IV

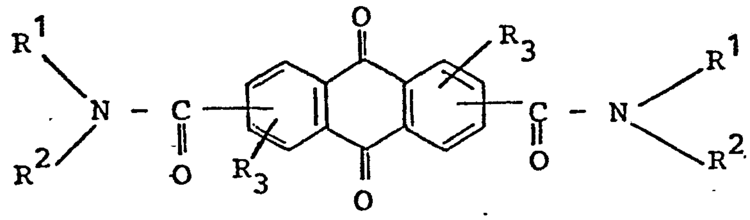

(IV)

worin $R^1$ und $R^2$ die zu Formel I erwähnten Bedeutungen haben und $R^3$ für Wasserstoff steht, reduziert und die als Zwischenprodukt erhaltenen Anthrahydrochinon-Derivate zu Anthrachinon-Derivaten der Formel I oxydiert und gegebenenfalls durch Zugabe einer physiologisch verträglichen Säure in ein Salz überführt, oder

c) ein Anthrachinon der Formel V

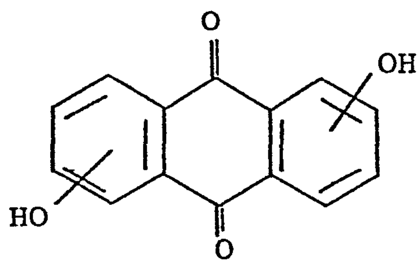

(V)

zu einem Anthrahydrochinon reduziert, anschließend mit Formaldehyd und einem Amin der Formel III umsetzt und das erhaltene Bis-aminomethyl-anthrahydrochinon-Derivat zu einem Anthrachinon-Derivat der Formel I oxydiert, und dieses gegebenenfalls durch Zugabe einer physiologisch verträglichen Säure in ein Salz überführt.

3. Arzneimittel, gekennzeichnet durch einen Gehalt an oder bestehend aus einem Bis-(aminomethyl)-anthrachinon-derivat der Formel I in Anspruch 1.

4. Verwendung eines Bis-(aminomethyl)-anthrachinon-derivates der Formel I in Anspruch 1 zur Behandlung von Protozoenerkrankungen, Viruserkrankungen oder Krebserkrankungen.

## Patentanspruch für Österreich

Verfahren zu Herstellung von Bis-Aminomethyl-anthrachinon-Derivaten der Formel I

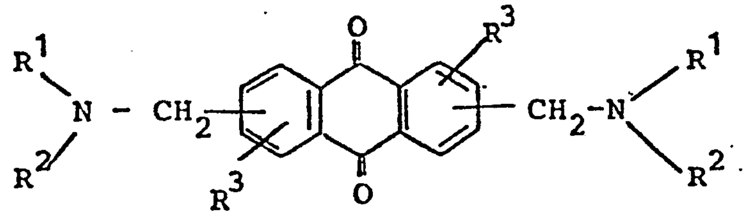

(I)

und ihrer Salze mit physiologisch verträglichen Säuren, worin $R^1$ und $R^2$ gleich oder verschieden sind und Wasserstoff, geradkettiges oder verzweigtes Alkyl mit ein bis vier Kohlenstoffatomen, oder $R^1$ und $R^2$ zusammen eine Alkylenkette mit vier, fünf oder sechs Kohlenstoffatomen, die mit dem Stickstoffatom einen fünf-, sechs- oder siebengliedrigen heterocyclischen Ring bilden, der ein weiteres Heteroatom aus der Gruppe Stickstoff, Sauerstoff und Schwefel enthalten kann und seinerseits durch geradkettiges oder verzweigtes Alkyl mit ein bis vier Kohlenstoffatomen substituiert sein kann, bedeuten, und worin $R^3$ für Wasserstoff oder die Hydroxylgruppe steht,
dadurch gekennzeichnet, daß man

a) ein substituiertes Dimethyl-anthrachinon der Formel II

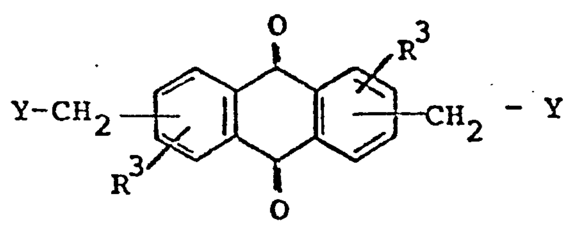

(II)

worin $R^3$ für Wasserstoff oder die Hydroxylgruppe steht und Y ein Halogenatom wie Chlor, Brom, Jod, eine Acyloxygruppe oder eine Arylsulfonyloxygruppe bedeutet, mit einem Amin der Formel III

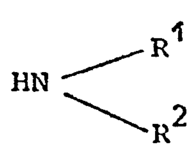

(III)

worin $R^1$ und $R^2$ die zu Formel I erwähnten Bedeutungen haben, umsetzt und gegebenenfalls durch Zugabe einer physiologisch verträglichen Säure in ein Salz überführt, oder

b) ein Anthrachinon-dicarbonsäureamid der Formel IV

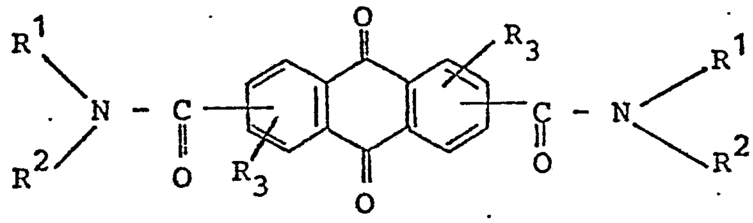

(IV)

worin $R^1$ und $R^2$ die zu Formel I erwähnten Bedeutungen haben und $R^3$ für Wasserstoff steht, reduziert und die als Zwischenprodukt erhaltenen Anthrachinon-Derivate zu Anthrachinon-Derivaten der Formel I oxydiert und gegebenenfalls durch Zugabe einer physiologisch verträglichen Säure in ein Salz überführt, oder

c) ein Anthrachinon der Formel V

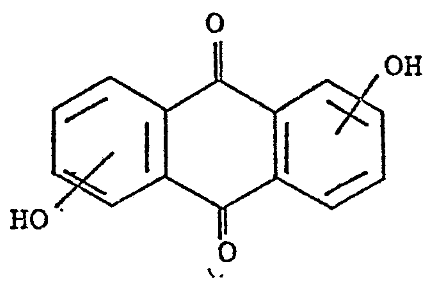

(V)

zu einem Anthrahydrochinon reduziert, anschließend mit Formaldehyd und einem Amin der Formel III umsetzt und das erhaltene Bis-aminomethyl-anthrahydrochinon-Derivat zu einem Anthrachinon-Derivat der Formel I oxydiert, und dieses gegebenenfalls durch Zugabe einer physiologisch verträglichen Säure in ein Salz überführt.

Europäisches Patentamt

**EUROPÄISCHER RECHERCHENBERICHT**

0052853
Nummer der Anmeldung

EP 81109738.5

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int Cl 3) |
|---|---|---|---|
| | DE - A1 - 2 827 197 (BAYER AG)<br>+ Seite 5, Zeilen 1-6 +<br>--<br>DE - A - 2 055 111 (BREDERECK, KARL, DR.)<br>+ Seite 1; Seite 2, Zeilen 1-3 +<br>--<br>AT - B - 359 484 (AMERICAN CYANAMID COMPANY) (10-11-1980)<br>+ Anspruch 1; Seite 14, Zeilen 22-24; Seite 15 +<br>---- | 1<br><br><br>1<br><br><br><br>1,3,4 | C 07 C 97/24<br>C 07 D 295/10<br>C 07 D 207/08<br>C 07 D 211/32<br>C 07 D 265/30<br>A 61 K 31/135<br>A 61 K 31/40<br>A 61 K 31/445<br>A 61 K 31/495<br>A 61 K 31/535<br>A 61 K 31/55<br><br>RECHERCHIERTE SACHGEBIETE (Int. Cl.3)<br>C 07 C 97/00<br>C 07 D 295/00<br>C 07 D 207/00<br>C 07 D 211/00<br>C 07 D 265/00<br><br>KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 11-01-1982 | HEIN |